(19)

Europäisches Patentamt

European Patent Office

Office européen des brevets

(11) **EP 3 586 878 A1**

(12) **EUROPÄISCHE PATENTANMELDUNG**

(43) Veröffentlichungstag:
**01.01.2020 Patentblatt 2020/01**

(51) Int Cl.:
***A61K 49/10*** *(2006.01)*

(21) Anmeldenummer: **18180099.6**

(22) Anmeldetag: **27.06.2018**

(84) Benannte Vertragsstaaten:
**AL AT BE BG CH CY CZ DE DK EE ES FI FR GB
GR HR HU IE IS IT LI LT LU LV MC MK MT NL NO
PL PT RO RS SE SI SK SM TR**
Benannte Erstreckungsstaaten:
**BA ME**
Benannte Validierungsstaaten:
**KH MA MD TN**

(71) Anmelder: **Charité - Universitätsmedizin Berlin
10117 Berlin (DE)**

(72) Erfinder:
• **Schellenberger, Eyk
10115 Berlin (DE)**

• **Hauptmann, Ralf
12203 Berlin (DE)**
• **Häckel, Akvile
06886 Lutherstadt Wittenberg (DE)**
• **Xie, Jing
10625 Berlin (DE)**
• **Schnorr, Jörg
16515 Oranienburg (DE)**
• **Hamm, Bernd
12207 Berlin (DE)**

(74) Vertreter: **Gulde & Partner
Patent- und Rechtsanwaltskanzlei mbB
Wallstraße 58/59
10179 Berlin (DE)**

(54) **VERFAHREN ZUR HERSTELLUNG EINES FE-TCDTA-KONTRASTMITTELS UND NACH DEM VERFAHREN ERHÄLTLICHES PRODUKT**

(57) Es wird ein Verfahren bereitgestellt, bei dem das anfallende Fe-*t*CDTA-Kontrastmittel eine verringerte Osmolalität aufweist. Das erfindungsgemäße Verfahren umfasst dazu die Schritte:

a) Herstellen einer wässrigen Lösung aus *t*CDTA und FeO(OH), wobei *t*CDTA und FeO(OH) in einem molaren Verhältnis von 1 : 1 bis 1 : 1,4 vorliegt;

b)

b.1) Einstellen eines pH-Werts der wässrigen Lösung auf pH 2,5 bis pH 4,5 durch Zusatz einer Base, vorzugsweise von Meglumin, und Abtrennen des Niederschlags; oder

b.2) Ausfällen des Fe-*t*CDTA-Kontrastmittels aus der wässrigen Lösung durch Zusatz von Aceton, Abtrennen des Präzipitats und Herstellen einer wässrigen Lösung aus dem Präzipitat; und

c) Einstellen eines pH-Werts der wässrigen Lösung auf pH 6,5 bis pH 8,0 durch Zusatz einer Base, vorzugsweise von Meglumin, und Abtrennen des ausgefallenen FeO(OH).

EP 3 586 878 A1

## Beschreibung

Die Erfindung betrifft ein Verfahren zur Herstellung eines Fe-*t*CDTA-Kontrastmittels sowie ein nach dem Verfahren erhältliches Fe-*t*CDTA-Kontrastmittel.

## Technologischer Hintergrund

Niedermolekulare Gadolinium-basierte Kontrastmittel (GBCA) haben sich als Standard zur Verbesserung der Aussagekraft von Magnetresonanztomographie (MRT)-Untersuchungen in allen Bereichen der Medizin durchgesetzt. In Deutschland wurden 2015 über 10,8 Mio. Magnetresonanztomographie (MRT)-Untersuchen durchgeführt. Bei etwa einem Drittel dieser MRT-Untersuchungen werden dabei GBCA für T1-gewichtete Bildgebungssequenzen intravenös appliziert, wodurch verbesserte Diagnosen bei akuten und chronischen Entzündungen, Tumoren, Gefäßneubildungen usw. ermöglicht werden.

Freie Gadolinium-Ionen sind toxisch. Aufgrund ihres im Vergleich zu Calcium-Ionen chemisch ähnlichen Verhaltens können diese beispielsweise in der Leber und im Knochensystem eingebaut werden und über Jahre dort verbleiben. Da die Ionenradien von Calcium und Gadolinium ähnlich sind, kann Gadolinium als Calciumantagonist wirken und beispielsweise die Kontraktilität des Myokards und das Gerinnungssystem hemmen.

Obwohl Gadolinium-Ionen toxisch sind, wurden GBCA mit der Maßgabe zugelassen, dass sie in den zugelassenen Pharmaka fest in Komplexbildnern, beispielsweise DTPA (Diethylentriaminpentaessigsäure) und DOTA (1,4,7,10-Tetraazacyclododecan-1,4,7,10-tetraessigsäure), eingebunden sind und zusammen mit diesen sehr schnell und vollständig vor allem durch die Niere ausgeschieden werden.

Allerdings hat sich gezeigt, dass die Ausscheidung unter Umständen nicht vollständig erfolgt. So wird in jüngster Zeit ein großer Teil der Fälle der nephrogenen systemischen Fibrose als Folge der toxischen Wirkung unvollständig ausgeschiedener Gadolinium-Kontrastmittel angesehen, die Patienten mit eingeschränkter Nierenfunktion verabreicht wurden. Eine andere tierexperimentelle Studie zeigte, dass nach Gabe von GBCA in Hautbiopsien von Ratten Gadolinium noch nach einem Jahr nachgewiesen werden konnte (Pietsch H, Lengsfeld P, Jost G, Frenzel T, Hütter J, Sieber MA Long-term retention of gadolinium in the skin of rodents following the administration of gadolinium-based contrast agents. Eur Radiol 2009). Das Ausmaß der Gadolinium-Anreicherung erwies sich dabei abhängig von der Art der Komplexbildner zu sein, insbesondere der Stabilität der Gadolinium-Komplexe.

Besonders bei wiederholter Gabe von niedermolekularen Kontrastmitteln, wie sie beispielsweise zum Brusttumor-Screening oder zu Verlaufskontrollen bei multipler Sklerose notwendig sind, wurden Ablagerungen im Hirngewebe von gesunden Personen gefunden (siehe beispielsweise McDonald RJ, McDonald JS, Kalimes OF, etal. Intracranial Gadolinium Deposition after Contrast-enhanced MR Imaging. Radiology 2015;275(3):772-782). Mittels ICP-MS wurden in Proben von verstorbenen Patienten auch Gadolinium-Ablagerungen von stabileren makrozyklischen GBCA besonders in Knochen und im Hirn gefunden (Murata N, Gonzalez-Cuyar LF, Murata K, Fligner C, Dills R, Hippe D, Maravilla KR Macrocyclic and Other Non-Group 1 Gadolinium Contrast Agents Deposit Low Levels of Gadolinium in Brain and Bone Tissue: Preliminary Results From 9 Patients With Normal Renal Function. Invest Radiol 2016; 51(7):447-453).

In der Vergangenheit konnte gezeigt werden, dass niedermolekulare Eisenkomplexe (besonders Fe-DTPA, Fe-*t*CDTA (*t*CDTA = trans-Cyclohexandiamintetraessigsäure) als Kontrastmittel für die MRT genutzt werden können. Die Relaxivitäten dieser Eisenkomplexe sind zwar im Vergleich zu den GBCA geringer, aber durch höhere Dosierungen lässt sich dieser Nachteil kompensieren (siehe beispielsweise White, Ramos, Huberty R, Brasch R, Engelstad B IRON (III) COMPLEXES AS MRI CONTRAST AGENTS. Proc. Int. Soc. Magn. Reson. Med. 1985;1985(S2):906-909). Aufgrund der großen Menge an körpereigenem Eisen (ca. 4 g für einen Erwachsenen) und der dafür vorhandenen Systeme für Aufnahme, Speicherung und Transport ist davon auszugehen, dass die Langzeittoxizität für Eisenkomplexe geringer sein sollte als für GBCA. Außerdem werden Eisenoxidnanopartikel (Kontrastmittel, Eisenersatztherapie), die im Gegensatz zu den fast vollständig ausgeschiedenen niedermolekularen Kontrastmitteln, nach i.v.-Gabe nahezu vollständig im Körper verbleiben, allgemein gut vertragen.

Es wurde bereits gezeigt, dass Eisenkomplexe sich auch für typische aktuelle Anwendungen, wie z. B. die Dynamische kontrastmittelgestützte MRT (DCE-MRI) eignen, die beispielsweise in der Brusttumordiagnostik eingesetzt wird (Boehm-Sturm P, Haeckel A, Hauptmann R, Mueller S, Kuhl OK, Schellenberger EA Low-Molecular-Weight Iron Chelates May Be an Alternative to Gadolinium-based Contrast Agents for T1-weighted Contrast-enhanced MR Imaging. Radiology 2017;170116).

US 5,362,475 A beschreibt unter anderem die Herstellung Eisen-basierter Kontrastmittel, konkret dem Natrium- oder N-Methylglucaminsalz eines Eisen(III)komplexes mit tCDTA-Ligand. Der in der Patentschrift beschriebene Syntheseweg sieht eine direkte Fällung der Eisen(III)Komplexe aus einer wässrigen $FeCl_3$-Lösung vor. Nachteilig ist, dass aufgrund des eingesetzten $FeCl_3$, Fe-*t*CDTA mit einem hohen Gehalt an Chloridionen anfällt, deren Abtrennung vom Eisen(III)komplex technisch aufwendig ist und nur ungenügend gelingt. Dies führt nach Neutralisation zu einer sehr hohen Salzkonzentration und damit hohen Osmolalität, die ungünstig für intravenöse Injektionen ist.

# EP 3 586 878 A1

**Zusammenfassung der Erfindung**

Erfindungsgemäß wird nunmehr ein Herstellungsverfahren bereitgestellt, bei dem das anfallende Fe-*t*CDTA-Kontrastmittel eine wesentlich verringerte Osmolalität aufweist. Das erfindungsgemäße Verfahren umfasst dazu die Schritte:

a) Herstellen einer wässrigen Lösung aus *t*CDTA und FeO(OH), wobei *t*CDTA und FeO(OH) in einem molaren Verhältnis von 1 : 1 bis 1 : 1,4, vorzugsweise 1 : 1,1 bis 1 : 1,3, vorliegt;

b)

b.1) Einstellen eines pH-Werts der wässrigen Lösung auf pH 2,5 bis pH 4,5 durch Zusatz einer Base, vorzugsweise von Meglumin, und Abtrennen des Niederschlags; oder

b.2) Ausfällen des Fe-*t*CDTA-Kontrastmittels aus der wässrigen Lösung durch Zusatz eines mit Wasser mischbaren organischen Lösungsmittels, Abtrennen des Präzipitats und Herstellen einer wässrigen Lösung aus dem Präzipitat; und

c) Einstellen eines pH-Werts der wässrigen Lösung auf pH 6,5 bis pH 8,0, vorzugsweise pH 7 bis pH 7,5, durch Zusatz einer Base, vorzugsweise von Meglumin, und Abtrennen des ausgefallenen FeO(OH).

Der Erfindung liegt die Erkenntnis zugrunde, dass sich ein Fe-*t*CDTA-Kontrastmittel geringer Osmolalität durch die spezifische Abfolge der Verfahrensschritte a) bis c) erhalten lässt.

Im Schritt a) wird zunächst eine wässrige Lösung aus dem sauren, mehrzähnigen Liganden *t*CDTA und in saurer Umgebung löslichem Eisen(III)-hydroxidoxid FeO(OH) hergestellt.

Vorzugsweise wird dabei die wässrige Lösung aus *t*CDTA-Lösung und FeO(OH) im Schritt a) nach der vollständigen Zugabe der Komponenten noch für 1 bis 3 h auf 70 bis 100°C, insbesondere 90 bis 100 °C erhitzt. Ein pH-Wert der wässrigen Lösung wird dabei vorzugsweise auf pH 0,1 bis pH 2 eingestellt. Der bevorzugte pH-Wertebereich der wässrigen Lösung kann schon durch entsprechende Vorgabe der Konzentration des sauren Liganden *t*CDTA erreicht werden.

Das in Schritt a) eingesetzte FeO(OH) wird vorzugsweise vorab frisch hergestellt und zwar nach zwei alternativen Varianten durch Fällung i) aus einer wässrigen $Fe(NO_3)_3$-Lösung unter Zusatz einer $NH_4OH$-Lösung oder ii) aus einer wässrigen $FeCl_3$-Lösung unter Zusatz einer NaOH-Lösung. Das nach diesen Varianten frisch hergestellte FeO(OH) lässt sich im Gegensatz zu kommerziell erhältlichen Produkten nahezu vollständig in saurer Umgebung auflösen.

Nach einer ersten Variante wird anschließend im Schritt b) ein pH-Wert der wässrigen Lösung auf pH 2,5 bis pH 4,5, vorzugsweise pH 3,5 bis pH 4,5, durch Zusatz einer Base, zum Beispiel NaOH und vorzugsweise von Meglumin, eingestellt und der gegebenenfalls entstehende Niederschlag abgetrennt. Es hat sich gezeigt, dass sich im Gegensatz zum niedrigen Ausgangs-pH unter diesen Bedingungen ein relativ stabiler Fe-*t*CDTA-Komplex vorgeformt wird, wodurch weniger Eisen in Form von FeO(OH) der Reaktion bei nachfolgenden Neutralisation entzogen wird. Durch diesen Zwischenschritt werden durch Verminderung von eisenfreiem *t*CDTA der Salzgehalt und somit auch die Osmolalität des Fe-*t*CDTA-Kontrastmittels nach Neutralisation stark gemindert. Vorzugsweise wird die wässrige Lösung aus *t*CDTA-Lösung und FeO(OH) nach dem Einstellen des pH-Wertes noch für 0,5 h oder länger, insbesondere 0,5 bis 2 h, bei Raumtemperatur gerührt.

Nach einer zweiten Variante wird im Schritt b) Fe-*t*CDTA aus der wässrigen Lösung durch Zusatz eines mit Wasser mischbaren organischen Lösungsmittels, vorzugsweise durch Zusatz von Ethanol, Acetonitril oder Aceton, besonders bevorzugt durch Zusatz von Aceton, ausgefällt, das Präzipitat abgetrennt, getrocknet und anschließend aus dem Präzipitat wieder eine wässrige Lösung hergestellt. Überschüssiges FeO(OH) verbleibt nach dieser Variante also in Lösung und wird abgetrennt. Der salzarme und saure Niederschlag aus Fe-*t*CDTA wird zur weiteren Aufbereitung wieder in Wasser aufgelöst.

Im Schritt c) des Verfahrens wird durch Zusatz einer Base, zum Beispiel NaOH und vorzugsweise von Meglumin, ein pH-Wert der wässrigen Lösung auf pH 6,5 bis pH 8,0, vorzugsweise pH 7,1 bis pH 7,5, insbesondere pH 7,3 bis pH 7,4, eingestellt und ausgefallenes FeO(OH) abgetrennt. Aus der verbleibenden wässrigen Lösung kann dann das Kontrastmittel mit hohem Reinheitsgrad isoliert werden.

Das nach dem erfindungsgemäßen Verfahren erhältliche Fe-*t*CDTA-Kontrastmittel zeichnet sich durch einen hohen Reinheitsgrad und insbesondere geringen Salzgehalt aus. Das nach dem Verfahren erhältliche Fe-*t*CDTA-Kontrastmittel zeigt insbesondere eine Osmolalität bei 0,25 mmol/l in Wasser bezogen auf den Fe-Gehalt von kleiner als 700 mOsm/kg, insbesondere 300 - 700 mOsm/kg. Daher ist das Kontrastmittel besonders geeignet für die intravenöse Injektion, besonders auch in höheren Dosierungen.

Weitere bevorzugte Ausgestaltungen der Erfindung ergeben sich aus der nachfolgenden Beschreibung.

## Detaillierte Beschreibung der Erfindung

Die Herstellung eines Fe-*t*CDTA-Kontrastmittels wird nachfolgend in zwei Ausführungsbeispielen näher erläutert.

### A. Synthese ausgehend von einer Eisen(III)-nitrat-Lösung

20 g an Eisen(III)-nitrat Hexahydrat (Fe(NO$_3$)$_3$*6H$_2$O) wurden in 100 ml Wasser (dest.) gelöst und filtriert. Eine Mischung aus 50 ml Ammoniumhydroxid-Lösung (Ammoniakwasser, 28 bis 30 Gew.% in Wasser) und 50 ml Wasser (dest.) wurde tropfenweise bei Raumtemperatur und unter Rühren der Fe(NO$_3$)$_3$-Lösung zugesetzt. Das entstandene unlösliche Eisen(III)-hydroxidoxid FeO(OH) wurde durch Filtration (Büchner-Trichter) gesammelt und mehrmals mit Wasser gewaschen.

17,1 g *t*CDTA wurde in 40 ml Wasser (dest.) gelöst. Das FeO(OH) wurde hinzugegeben und das Volumen der Lösung auf 100 ml mit Wasser (dest.) aufgefüllt. Die Lösung wurde ca. 2 h bei 95 °C erhitzt und dabei gerührt. Danach wurde die Lösung abgekühlt, filtriert und das Volumen durch Erhitzen auf 20 ml reduziert. Aus der reduzierten Lösung wurden die Eisenkomplexe durch Zugabe von Azeton (ca. 300 ml) ausgefällt, der Niederschlag zentrifugiert und getrocknet.

Der Niederschlag aus Fe-*t*CDTA wurde erneut in Wasser (dest.) gelöst und der pH mit Meglumin auf 7,3 bis 7,4 eingestellt, wobei eine relative Eisenkonzentration der Lösung 38 mg/ml betrug.

### B. Synthese ausgehend von einer Eisen(III)-chlorid-Lösung

Zu einer Lösung von 18,92 g Eisen(III)-chlorid Hexahydrat (FeCl$_3$*6H$_2$O) in 70 ml Wasser (dest.) wurde tropfenweise eine Lösung von 14 g NaOH in 70 ml Wasser (dest.) unter Rühren zugesetzt. Das ausfallende Eisen(III)-hydroxidoxid FeO(OH) wurde abgetrennt (Zentrifugation 3 min bei 1000 x g) und 3 Mal mit Wasser (dest.) gewaschen.

18,22 g *t*CDTA wurde in 50 ml Wasser (dest.) suspendiert und zum FeO(OH) gegeben. Die Mischung wurde unter Rühren für 2 h auf 95 °C erhitzt und nach dem Abkühlen zentrifugiert (3 min bei 1000 x g) und über einen 0,45 $\mu$m Spritzenfilter filtriert.

Der pH der erhaltenen Lösung wurde mit Meglumin auf 4 eingestellt und 1 h bei Raumtemperatur gerührt. Gegebenenfalls anfallende Niederschläge werden durch Filtration abgetrennt.

Nach 1 Stunde wurde der pH-Wert mit Meglumin auf 7,4 eingestellt und der entsprechende Niederschlag abzentrifugiert und filtriert.

Innerhalb der ersten 1 bis 3 Tage bildete sich FeO(OH)-Niederschlag. Die Lösung wurde für weitere 12h bei Raumtemperatur stehen gelassen. Die tCDTA-Lösung wurde zentrifugiert (3 min bei 1000 x g), dann vom Eisen(III)-hydroxid-Präzipitat abgenommen und danach mit einem 0,2 $\mu$m Spritzenfilter filtriert. Die Lösung wurde nach 3 Tagen nochmals zentrifugiert (3 min bei 1000 x g) um noch vorhandene Spuren von Eisen(III)-hydroxid abzutrennen.

### Bestimmung der Osmolalität

Die Osmolalität gibt die Molalität der osmotisch aktiven Teilchen in einer Lösung an:

$$b_{osm} = \frac{n_{osm}}{m_{sol}}$$

mit

$n_{osm}$: Stoffmenge osmotisch aktiver Teilchen
$m_{sol}$: Masse des Lösungsmittels, hier Wasser

Die Osmolalität gibt hier demnach die Teilchenanzahl der osmotisch aktiven Substanzen pro Kilogramm Wasser an und wurde wie folgt bestimmt:
Für die Osmolalitätsmessungen wurde das Gefrierpunkt-Messgerät OSMOMAT 030 von Gonotec GmbH benutzt. Zur Bestimmung der Gesamt-Osmolalität in wässrigen Lösungen wurden vergleichende Messungen der Gefrierpunkte von reinem Wasser und Lösungen durchgeführt. Während Wasser einen Gefrierpunkt von 0 °C besitzt, zeigt eine Lösung mit einer Salzkonzentration von 1 Osmol/kg einen Gefrierpunkt von -1,858 °C. Das bedeutet, dass ein Mol einer gegebenen nicht-dissoziierten Substanz (6.023 x 1023 Teile verdünnt in einem Kilogramm Wasser) senkt den Gefrierpunkt von einer Lösung um 1,858 ° C. Das Gerät nutzt die folgenden Definitionen zur Berechnung der Osmolalität: $C_{osm} = \Delta T / K$ mit $C_{osm}$ = Osmolalität [osmol/kg], T = Gefrierpunkt-Absenkung [°C], K = 1.858°C kg/osmol Gefrierpunktkonstante.

Der nachfolgenden Tabelle 1 ist die nach dieser Methode bestimmte Osmolalität der beiden Produkte der Ausführungsbeispiele. Zum Vergleich wurde in gleicher Weise die Osmolalität des gängigen Gadolinium-Kontrastmittels Magnevist bestimmt. Ferner wurde Fe-*t*CDTA entsprechend der in US 5,362,475 A genannten Vorschrift synthetisiert und die Osmolalität bestimmt.

**Tabelle 1**

| Osmolalität [mOsm/kg] bei 0,25 mmol/l in Wasser (c bezogen auf Fe bzw. Gd) | |
|---|---|
| Magnevist (Gadopentetat-Dimeglumin, Gd-DTPA) | 815 |
| Synthese Fe-*t*CDTA nach US 5,362,475 A | 3030 |
| Fe-*t*CDTA-Kontrastmittel nach Syntheseweg **A** | 517 |
| Fe-*t*CDTA-Kontrastmittel nach Syntheseweg **B** | 398 |

Wie ersichtlich, ist die Osmolalität der erfindungsgemäß erhältlichen Fe-*t*CDTA-Kontrastmittel stark gemindert, so dass sie sich in besonderer Weise für die intravenöse Injektion eignen.

**Orientierende Toxizitätsbestimmungen**

In ersten orientierenden Untersuchungen mit Fe-*t*CDTA-Kontrastmittel nach Syntheseweg **A,** das Ratten intravenös appliziert wurde, haben die Tiere Dosen von 2 mmol pro kg Körpergewicht (10-fache Dosis zur typischen Anwendungsdosis von 0,2 mmol pro kg) sehr gut vertragen. Die Urinwerte waren weitestgehend normal. Das Fe-*t*CDTA-Kontrastmittel nach Syntheseweg **B** wurde sogar mit 4 mmol pro kg Körpergewicht (20-fache Dosis zur typischen Anwendungsdosis) appliziert, was etwa 11 g Eisen bei einem Menschen mit 50 kg entspricht. Die Ratten haben diese Dosis gut toleriert und lediglich nach einem und zwei Tagen erhöhte Proteinwerte im Urin gezeigt. Bei der Nachkontrolle nach 14 Tagen waren alle Werte normal.

**Patentansprüche**

1. Verfahren zur Herstellung eines Fe-*t*CDTA-Kontrastmittels, umfassend die Schritte:

    a) Herstellen einer wässrigen Lösung aus *t*CDTA und FeO(OH), wobei *t*CDTA und FeO(OH) in einem molaren Verhältnis von 1 : 1 bis 1 : 1,4 vorliegt;
    b)

    b.1) Einstellen eines pH-Werts der wässrigen Lösung auf pH 2,5 bis pH 4,5 durch Zusatz einer Base, vorzugsweise von Meglumin, und Abtrennen des Niederschlags; oder
    b.2) Ausfällen von Fe-*t*CDTA aus der wässrigen Lösung durch Zusatz eines mit Wasser mischbaren organischen Lösungsmittels, Abtrennen des Präzipitats und Herstellen einer wässrigen Lösung aus dem Präzipitat; und

    c) Einstellen eines pH-Werts der wässrigen Lösung auf pH 6,5 bis pH 8,0 durch Zusatz einer Base, vorzugsweise von Meglumin, und Abtrennen des ausgefallenen FeO(OH).

2. Verfahren nach Anspruch 1, bei dem das in Schritt a) eingesetzte FeO(OH) vorab hergestellt wird durch Fällung i) aus einer wässrigen Fe(NO$_3$)$_3$-Lösung unter Zusatz einer NH$_4$OH-Lösung oder ii) aus einer wässrigen FeCl$_3$-Lösung unter Zusatz einer NaOH-Lösung.

3. Verfahren nach Anspruch 1 oder 2, bei dem die wässrige Lösung aus *t*CDTA-Lösung und FeO(OH) im Schritt a) nach der vollständigen Zugabe der Komponenten noch für 1 bis 3 h auf 70 bis 100 °C erhitzt wird.

4. Verfahren nach einem der vorhergehenden Ansprüche, bei dem ein pH-Wert der wässrigen Lösung im Schritt a) auf pH 0,1 bis pH 2 eingestellt wird.

5. Verfahren nach einem der vorhergehenden Ansprüche, bei dem die wässrige Lösung aus *t*CDTA-Lösung und FeO(OH) im Schritt b.1) nach dem Einstellen des pH-Wertes noch für 0,5 h oder länger bei Raumtemperatur gerührt wird.

6. Verfahren nach einem der vorhergehenden Ansprüche, bei dem im Schritt b.2) das Ausfällen von Fe-*t*CDTA aus der wässrigen Lösung durch Zusatz von Aceton erfolgt.

7. Fe-*t*CDTA-Kontrastmittel, hergestellt nach dem Verfahren gemäß Anspruch 1, wobei eine Osmolalität des Fe-*t*CDTA-Kontrastmittels bei 0,25 mmol/l in Wasser bezogen auf den Fe-Gehalt kleiner als 700 mOsm/kg ist.

**Europäisches Patentamt**
**European Patent Office**
**Office européen des brevets**

## EUROPÄISCHER RECHERCHENBERICHT

Nummer der Anmeldung

EP 18 18 0099

### EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| A,D | US 5 362 475 A (GRIES HEINZ [DE] ET AL) 8. November 1994 (1994-11-08) * Beispiel 7 * ----- | 1-7 | INV. A61K49/10 |
| A | PHILIPP BOEHM-STURM ET AL: "Low-Molecular-Weight Iron Chelates May Be an Alternative to Gadolinium-based Contrast Agents for T1-weighted Contrast-enhanced MR Imaging", RADIOLOGY, Bd. 286, Nr. 2, 1. Februar 2018 (2018-02-01), Seiten 537-546, XP055524104, US ISSN: 0033-8419, DOI: 10.1148/radiol.2017170116 & PHILIPP BOEHM-STURM ET AL: "Low-Molecular-Weight Iron Chelates May Be an Alternative to Gadolinium-based Contrast Agents for T1-weighted Contrast-enhanced MR Imaging, Appendix E1", RADIOLOGY, Bd. 286, Nr. 2, 1. Februar 2018 (2018-02-01), Seiten 1-2, XP055524745, US ISSN: 0033-8419, DOI: 10.1148/radiol.2017170116 * Seite 1, Absatz 4 * ----- | 1-7 | RECHERCHIERTE SACHGEBIETE (IPC) A61K |

Der vorliegende Recherchenbericht wurde für alle Patentansprüche erstellt

| Recherchenort | Abschlußdatum der Recherche | Prüfer |
|---|---|---|
| Den Haag | 19. November 2018 | Langer, Miren |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument

................................................................
& : Mitglied der gleichen Patentfamilie, übereinstimmendes Dokument

EPO FORM 1503 03.82 (P04C03)

## ANHANG ZUM EUROPÄISCHEN RECHERCHENBERICHT
## ÜBER DIE EUROPÄISCHE PATENTANMELDUNG NR.

EP 18 18 0099

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

19-11-2018

| Im Recherchenbericht angeführtes Patentdokument | Datum der Veröffentlichung | Mitglied(er) der Patentfamilie | Datum der Veröffentlichung |
|---|---|---|---|
| US 5362475 A | 08-11-1994 | US 4957939 A<br>US 4963344 A<br>US 5021236 A<br>US 5362475 A<br>US 5595725 A | 18-09-1990<br>16-10-1990<br>04-06-1991<br>08-11-1994<br>21-01-1997 |

EPO FORM P0461

Für nähere Einzelheiten zu diesem Anhang : siehe Amtsblatt des Europäischen Patentamts, Nr.12/82

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- US 5362475 A

**In der Beschreibung aufgeführte Nicht-Patentliteratur**

- **MCDONALD RJ ; MCDONALD JS ; KALIMES OF et al.** Intracranial Gadolinium Deposition after Contrast-enhanced MR Imaging. *Radiology,* 2015, vol. 275 (3), 772-782
- **MURATA N ; GONZALEZ-CUYAR LF ; MURATA K ; FLIGNER C ; DILLS R ; HIPPE D ; MARAVILLA KR.** Macrocyclic and Other Non-Group 1 Gadolinium Contrast Agents Deposit Low Levels of Gadolinium in Brain and Bone Tissue: Preliminary Results From 9 Patients With Normal Renal Function. *Invest Radiol,* 2016, vol. 51 (7), 447-453
- **WHITE ; RAMOS ; HUBERTY R ; BRASCH R.** Engelstad B IRON (III) COMPLEXES AS MRI CONTRAST AGENTS. *Proc. Int. Soc. Magn. Reson. Med. 1985,* 1985, vol. S2, 906-909
- **BOEHM-STURM P ; HAECKEL A ; HAUPTMANN R ; MUELLER S ; KUHL OK ; SCHELLENBERGER EA.** Low-Molecular-Weight Iron Chelates May Be an Alternative to Gadolinium-based Contrast Agents for T1-weighted Contrast-enhanced MR Imaging. *Radiology,* 2017, 170116